# EUROPEAN PATENT APPLICATION

(11) **EP 0 749 978 A1**
(43) Date of publication of application: **27.12.1996**
(21) Application number: 95906536.8
(22) Date of filing: 27.01.1995
(51) Int. Cl.: C07K 5/078, C07K 1/14

(54) **METHOD OF SEPARATING AND PURIFYING TRH ANALOGS AND PROCESS FOR PRODUCING SOLVATES OF TRH ANALOGS**

(30) Priority: 31.01.1994 JP 28816/94
(71) Applicant: Japan Tobacco Inc., Minato-Ku Tokyo 105 (JP)
(72) Inventor: SAKUMA, Kazuhiko, Central Pharmaceutical Research, Takatsuki-shi, Osaka 569 (JP); HARA, Katsuyoshi, Central Pharmaceutical Research, Takatsuki-shi, Osaka 569 (JP); HARUTA, Junichi, Central Pharmaceutical Research, Takatsuki-shi, Osaka 569 (JP); SAITOH, Akira, Central Pharmaceutical Research, Takatsuki-shi, Osaka 569 (JP); UCHIDA, Itsuo, Central Pharmaceutical Research, Takatsuki-shi, Osaka 569 (JP)
(74) Representative: von Kreisler, Alek, Dipl.-Chem.
(86) International application number: JP9500107
(87) International publication number: WO9520598

(57) **Abstract**

A method for separation and purification of a TRH derivative, which is characterized by introducing an imino-protecting group into the histidine moiety of a TRH analog to give an imino-protected compound of the following formula [II] 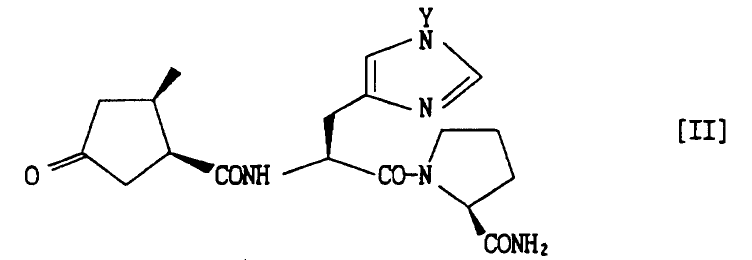 wherein Y is an imino-protecting group, and separating-purifying same in an organic solvent. According to the present invention, compound [I] can be separated and purified efficiently from the reaction mixture by a simple and easy method. The compound [I] thus obtained can be recrystallized with ease from water and the like as a solvate, thereby permitting easy production of solvate [III] at high purity and in high yields.

## Description

### Technical Field

The present invention relates to a method for separation and purification of a TRH analog useful as a pharmaceutical product, specifically a therapeutic agent for central nervous disorders, which exhibits stronger activity than TRH (thyrotropin releasing hormone) or a derivative thereof in central nerve activating action as observed in body temperature elevating action, spontaneous hyperkinetic action and ipsilateral reflex increasing action, and to a method for producing a solvate thereof.

### Background Art

As a compound related to the compound [I] to be separated and purified according to the method of the present invention, there has been known L-pyroglutamyl-L-histidyl-L-prolineamide (pGlu-His-Pro-NH₂) which is called TRH (thyrotropin releasing hormone). In addition to the conventionally-known TSH (thyrotropin: thyroid-stimulating hormone) releasing activity, TRH has been known to be useful as a therapeutic agent for consciousness disorders caused by cerebral function disorders.

With respect of the present invention, the present inventors have found a TRH analog of the following formula [I] 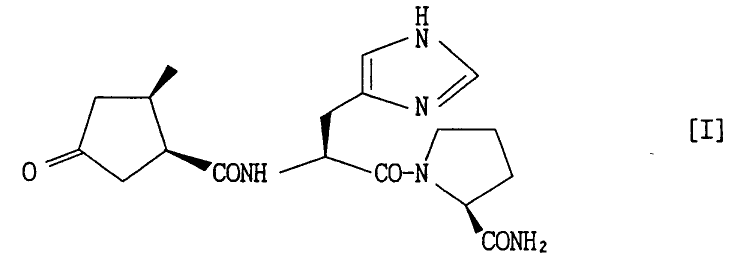 which has stronger central activating action and longer duration than known TRH derivatives, not to mention TRH, and which is useful as a therapeutic agent for central nervous disorders, and filed a patent application directed thereto (Japanese Patent Unexamined Publication No. 236397/1991).

However, the process described in Japanese Patent Unexamined Publication No. 236397/1991 requires use of separation columns and very large amounts of solvents in the purification step, and is unsuitable for industrial production.

### Disclosure of the Invention

The present inventors have conducted intensive studies in an attempt to overcome such defects of the conventional method, and found that once protecting a histidine moiety of a TRH analog with an imino-protecting group, and separating in this state with an organic solvent permits facilitated and efficient separation and purification of the TRH analog, and further that such imino-protecting group can be easily eliminated and the deprotected compound can be easily recrystallized as a solvate from water or lower alcohol without specific purification, which resulted in the completion of the present invention.

Accordingly, the present invention provides the following (1) to (4).
(1) A method for separation and purification of a TRH derivative, comprising introducing an imino-protecting group into a histidine moiety of a TRH analog of the following formula [I] 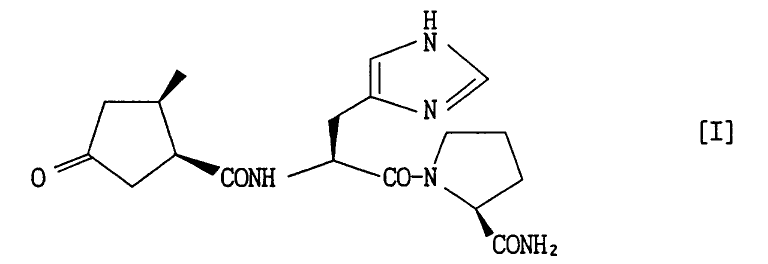 to give an imino-protected compound of the following formula [II] 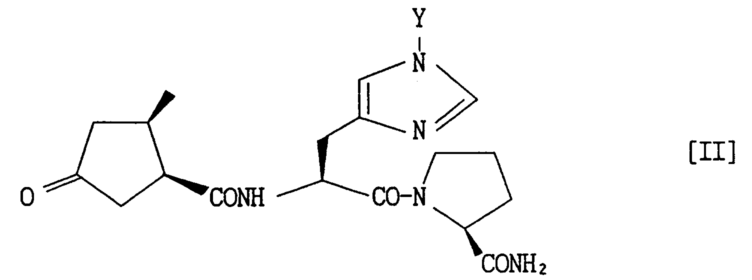 wherein Y is an imino-protecting group, and separating-purifying same in an organic solvent.
(2) The method of (1) above wherein the imino-protecting group is benzyloxycarbonyl group.
(3) The method of (1) above wherein the imino-protecting group is an acyl group.
(4) The method of (3) above wherein the acyl group is benzoyl group.
(5) A method for producing a solvate of a TRH derivative, comprising further recrystallizing, from water or a lower alcohol, a compound of the following formula [I] 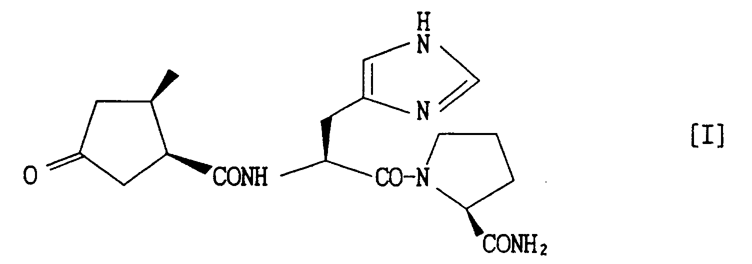 which is obtained by catalytic hydrogenation or solvolysis of the imino-protected compound [II] obtained in (1) above.
(6) Nα-[(1S,2R)-2-Methyl-4-oxocyclopentanecarbonyl]-L-histidyl-L-prolineamide hydrate obtained by the method of (5) above.

In the present invention, each symbol denotes the following.

The "imino-protecting group" means, for example, acyl such as acetyl, propionyl, butyryl, valeryl, palmitoyl, stearoyl, oxalyl, malonyl, succinyl, benzoyl, o-nitrobenzoyl, m-nitrobenzoyl, p-nitrobenzoyl, toluoyl, salicyloyl and furoyl, tert-butyloxycarbonyl, benzyloxycarbonyl, p-toluenesulfonyl or methanesulfonyl group, with preference given to benzyloxycarbonyl and benzoyl groups.

The "lower alcohol" means straight or branched alcohol having 1 to 4 carbon atoms. Specific examples include methanol, ethanol, n-propanol, isopropanol, n-butanol, isobutanol, sec-butanol and tert-butanol, with preference given to ethanol.

A method for separation and purification of a TRH analog of the following formula [I] 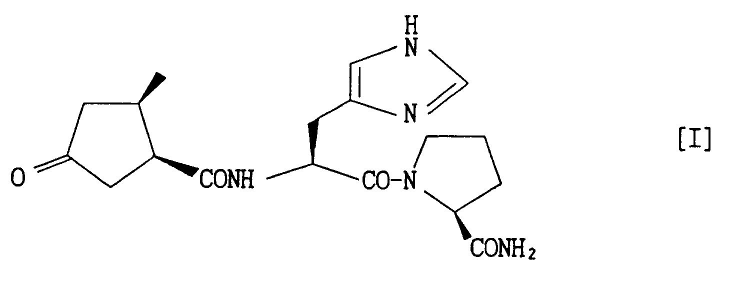 and a method for producing a solvate thereof are described in detail in the following.

A separation-purification and production flow chart is given below, wherein Y is an imino-protecting group, and R is a straight or branched lower alkyl having 1 to 4 carbon atoms, which is exemplified by methyl, ethyl, n-propyl, isopropyl, n-butyl, isobutyl, sec-butyl and tert-butyl group, with preference given to ethyl group.

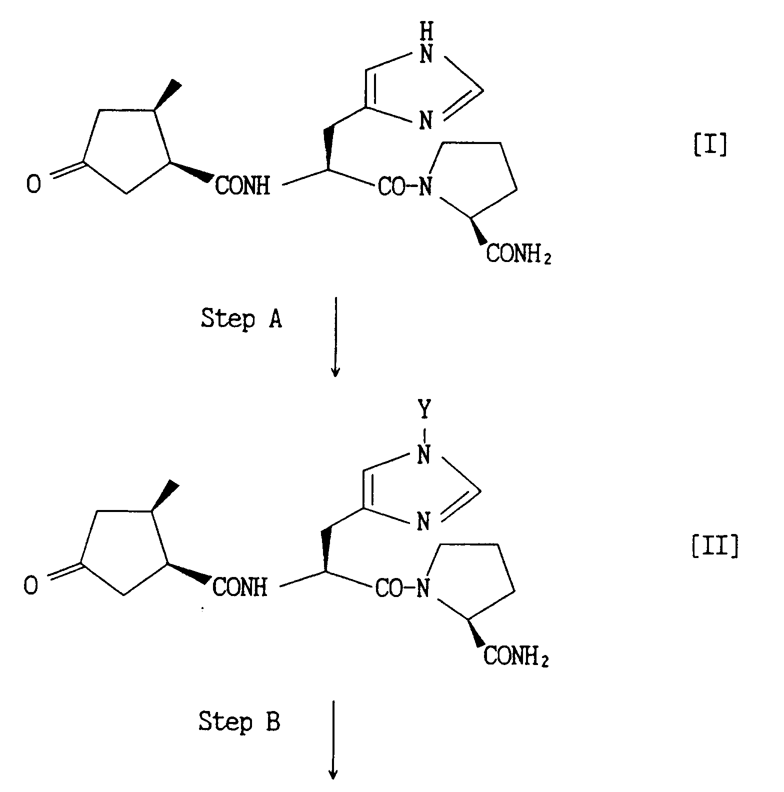

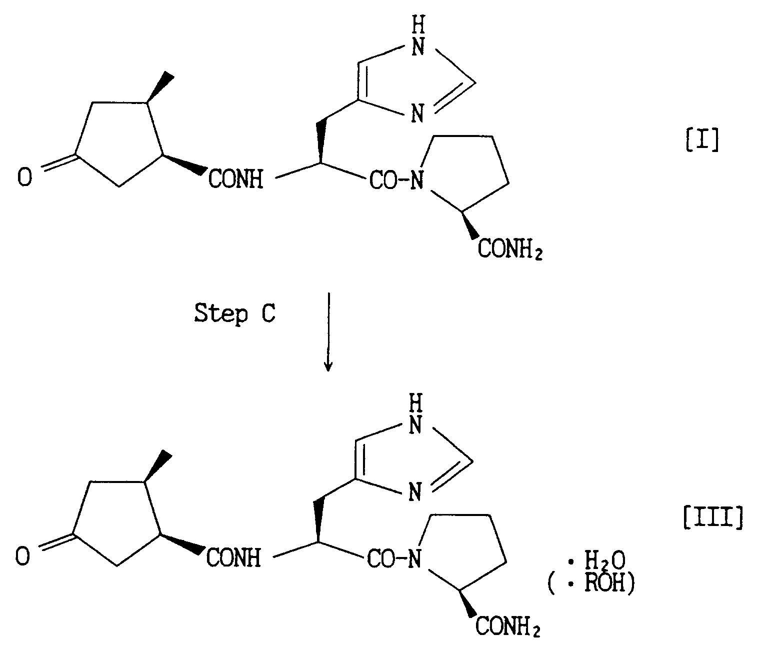

### [Step A] Protection of histidine moiety of TRH analog and separation-purification thereof

This step concerns protection of imidazole at the histidine moiety of a known TRH analog compound [I] (Japanese Patent Unexamined Publication No. 236397/1991) with an imino-protecting group to give a protected compound [II], which can be performed by a known method, such as reaction of compound [I] with benzoyl chloride, benzyloxycarbonyl chloride, and the like. The compound [I] obtained without purification by a production method conventionally known can be used. For example, the protection with an imino-protecting group can be carried out in a suitable solvent in the presence of a base. Examples of suitable imino-protecting group include acyl group such as acetyl, propionyl, butyryl, valeryl, palmitoyl, stearoyl, oxalyl, malonyl, succinyl, benzoyl, o-nitrobenzoyl, m-nitrobenzoyl, p-nitrobenzoyl, toluoyl, salicyloyl and furoyl, tert-butyloxycarbonyl, benzyloxycarbonyl, p-toluenesulfonyl and methanesulfonyl groups, with preference given to benzyloxycarbonyl and benzoyl groups.

The reaction temperature is suitably from -40°C to 40°C, preferably from -10°C to 10°C.

Examples of adequate base include trialkylamines such as triethylamine and trimethylamine; N,N-dialkylanilines such as N,N-dimethylaniline and N,N-diethylaniline; pyridine and dimethylamino-pyridine; and N-alkylmorpholines such as N-methylmorpholine; alkali metal hydroxides such as potassium hydroxide and sodium hydroxide; alkali metal carbonates such as potassium carbonate; and alkali metal hydrogencarbonates such as sodium hydrogencarbonate, with preference given to triethylamine.

The reaction solvent used in Step A is, for example, dimethylformamide, methylene chloride, dioxane, tetrahydrofuran, acetonitrile, ethyl acetate, pyridine, acetone or water, with preference given to dimethylformamide.

The imino-protected compound [II] thus obtained has an improved lipophilicity and can be separated and purified with extreme easiness by extraction using a suitable organic solvent. Examples of suitable organic solvent include dichloromethane, chloroform and ethyl acetate, with preference given to dichloromethane.

### [Step B] Elimination of imino-protecting group

The imino-protecting group of the imino-protected compound [II] obtained in the above Step A can be easily removed by a known method according to the kind of the imino-protecting group. For example, a catalytic hydrogenation or solvolysis can be applied.

The catalytic hydrogenation can be performed by, for example, hydrogenation of compound [II] prepared in Step A in a suitable solvent in the presence of a catalyst under hydrogen. The reaction solvent is, for example, lower alcohols such as methanol and ethanol; ether solvent such as tetrahydrofuran, dioxane, diethyl ether, diisopropyl ether, ethylene glycol dimethyl ether and diethylene glycol dimethyl ether, or a mixed solvent thereof. Preferred are lower alcohols and more preferred is methanol.

Examples of the catalyst for catalytic hydrogenation include palladium, palladium alumina and palladium-carbon. Preferred is palladium-carbon. The reaction temperature is suitably from 0°C to 50°C, preferably from 10°C to 30°C.

The solvolysis can be performed in a suitable solvent in the presence or absence of a suitable catalyst. Examples of suitable solvent include lower alcohol and water, preferably methanol, and examples of suitable catalyst include solid acid such as zeolite, silica gel and amberlite. Preferred is zeolite. The reaction temperature is suitably from 0°C to 50°C, preferably from 10°C to 30°C.

The catalytic hydrogenation is particularly suitable when the imino-protecting group is benzyloxycarbonyl, and solvolysis is suitable when the imino-protecting group is benzyloxycarbonyl or acyl group.

### [Step C] Preparation of solvate (recrystallization)

The objective compound [III] of the present invention can be recrystallized as a solvate by a general method. For example, it can be obtained by dissolving same in water or lower alcohol, and stirring or allowing to stand.

The present invention is explained in more detail in the following by illustrative examples. The symbols used in examples denote the following.
- NMR: nuclear magnetic resonance spectrum (¹H-NMR)
- WSC: 1-ethyl-3-(3-dimethylaminopropyl)-carbodiimide

Of the compounds to follow, the compounds having the symbol of N^{im} are mixtures of the compounds wherein the π nitrogen atom of the imidazole ring of histidine has been substituted as in 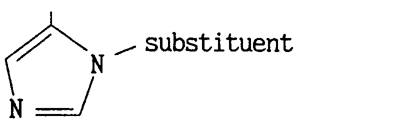 or the compounds wherein the τ nitrogen atom has been substituted as in

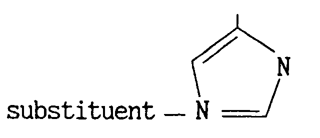

### Example 1

### Nα-[(1S,2R)-2-Methyl-4-oxocyclopentanecarbonyl]-N^{im}-benzyloxycarbonyl-L-histidyl-L-prolineamide (compound [II])

(1S,2R)-2-Methyl-4-oxocyclopentanecarboxylic acid (35.5 g) and N-hydroxybenzotriazole monohydrate (42.0 g) were dissolved in N,N'-dimethylformamide (500 ml), and ice-cooled. WSC · HCl (52.7 g) was added thereto. The mixture was stirred for 7 hours under ice-cooling, and His-Pro-NH₂ · 2HBr (124 g) was dissolved, followed by addition of triethylamine (92 ml). The mixture was stirred for 15 hours at 4°C to give a TRH analog [I].

This reaction mixture was ice-cooled, and triethylamine (45 ml) was added, followed by rather gradual addition of benzyloxycarbonyl chloride (35.7 ml). The mixture was stirred for 3 hours under ice-cooling. The reaction mixture was added to dichloromethane (500 ml) and washed with water (1000 ml), a saturated aqueous sodium hydrogencarbonate solution (400 ml × 1, 200ml × 1) and water (400 ml × 1, 200 ml × 1). The dichloromethane layer was dried over anhydrous magnesium sulfate, filtrated and concentrated under reduced pressure to give a residue (149 g). The residue was crystallized from acetone (350 ml) to give the title compound (74.2 g, yield 58%) as white powdery crystals.
mp : 102.1 - 104.3°C, [α]_{D} -49.0° (C=1.53, CHCl₃)
NMR (CDCl₃)δ ppm: 1.01(d,3H,J=7.89Hz), 1.85-2.43(m,7H), 2.44-2.71(m,2H), 2.94-3.10(m,2H), 3.14(dd,1H,J=7.75 and 14.2Hz), 3.32-3.43(m,1H), 3.61-3.72(m,1H), 4.58(dd,1H,J=3.51 and 8.35Hz), 4.85-4.95(m,1H), 5.41(d,2H,J=1.39Hz), 5.61(s,1H), 7.10(d,1H,J=7.34), 7.27(s,1H), 7.43(s,5H), 8.02(s,1H), 8.40(s,1H)

### Example 2

### Nα-[(1S,2R)-2-Methyl-4-oxocyclopentanecarbonyl]-L-histidyl-L-prolineamide · 1H₂O (compound [III])

Nα-[(1S,2R)-2-Methyl-4-oxocyclopentanecarbonyl]-N^{im}-benzyloxycarbonyl-L-histidyl-L-prolineamide (40.0 g) obtained in the same manner as in Example 1 was dissolved in methanol (500 ml), and 10% palladium-carbon (1.0 g) was added, followed by catalytic hydrogenation for 6 hours. The catalyst was filtered off on celite and the filtrate was concentrated under reduced pressure to give a residue (32.0 g). The residue was recrystallized from methanol(75 ml) and the obtained crystals (22.5 g) were further recrystallized from water (45 ml) to give the title compound (16.0 g, yield 51.8%) as white powdery crystals.
[α]_{D} -75.3° (C=1.02, CH₃OH)

| Elemental analysis: C₁₈H₂₅N₅O₄ · 1H₂O | | | |
|---|---|---|---|
| Calculated : | C, 54.95; | H, 6.92; | N, 17.80 |
| Found : | C, 55.20; | H, 6.76; | N, 17.77 |

### Example 3

### Nα-[(1S,2R)-2-Methyl-4-oxocyclopentanecarbonyl]-L-histidyl-L-prolineamide · 1H₂O (compound [III])

Nα-[(1S,2R)-2-Methyl-4-oxocyclopentanecarbonyl]-N^{im}-benzyloxycarbonyl-L-histidyl-L-prolineamide (4.0 g) obtained in the same manner as in Example 1 was dissolved in methanol (500 ml) and stirred at room temperature for 20 hours, which was followed by concentration under reduced pressure. The residue was partitioned between dichloromethane (30 ml) and water (50 ml), and the aqueous layer was separated. The aqueous layer was washed with dichloromethane (20 ml × 3) and concentrated under reduced pressure to give 3.0 g of a residue. The residue was recrystallized from methanol (8 ml) and the obtained crystals (2.3 g) were further recrystallized from water (8 ml) to give the title compound (1.53 g, yield 49.5%) as white powdery crystals.

### Example 4

### Nα-[(1S,2R)-2-Methyl-4-oxocyclopentanecarbonyl]-L-histidyl-L-prolineamide · 1CH₃OH (compound [III]; R=methyl)

Nα-[(1S,2R)-2-Methyl-4-oxocyclopentanecarbonyl]-N^{im}-benzyloxycarbonyl-L-histidyl-L-prolineamide (40.0 g) obtained in the same manner as in Example 1 was dissolved in methanol (500 ml) and 10% palladium-carbon (1.0 g) was added, followed by catalytic hydrogenation for 6 hours. The catalyst was filtered off on celite and the filtrate was concentrated under reduced pressure to give a residue (32.0 g). The residue was recrystallized from methanol (75 ml) to give Nα-[(1S,2R)-2-methyl-4-oxocyclopentanecarbonyl]-L-histidyl-L-prolineamide (22.5 g) which was further recrystallized from methanol (68 ml) to give the title compound (15.5 g).

| Elemental analysis: C₁₈H₂₅N₅O₄ · 1 CH₃OH | | | |
|---|---|---|---|
| Calculated : | C, 56.01; | H, 7.17; | N, 17.19 |
| Found : | C, 55.78; | H, 7.16; | N, 17.46 |

### Example 5

### Nα-[(1S,2R)-2-Methyl-4-oxocyclopentanecarbonyl]-L-histidyl-L-prolineamide · 1 C₂H₅OH (compound [III]; R=ethyl)

Nα-[(1S,2R)-2-Methyl-4-oxocyclopentanecarbonyl]-N^{im}-benzyloxycarbonyl-L-histidyl-L-prolineamide (40.0 g) obtained in the same manner as in Example 1 was dissolved in methanol (500 ml), and 10% palladium-carbon (1.0 g) was added, followed by catalytic hydrogenation for 6 hours. The catalyst was filtered off on celite and the filtrate was concentrated under reduced pressure to give a residue (32.0 g). The residue was recrystallized from methanol (75 ml) to give Nα-[(1S,2R)-2-methyl-4-oxocyclopentanecarbonyl]-L-histidyl-L-prolineamide (22.5 g) which was further recrystallized from ethanol (68 ml) to give the title compound (19.8 g).

| Elemental analysis: C₁₈H₂₅N₅O₄ · 1 C₂H₅OH | | | |
|---|---|---|---|
| Calculated : | C, 56.99; | H, 7.41; | N, 16.61 |
| Found : | C, 56.76; | H, 7.12; | N, 16.79 |

### Example 6

### Nα-[(1S,2R)-2-Methyl-4-oxocyclopentanecarbonyl]-L-histidyl-L-prolineamide · 1 i-C₃H₈OH (compound [III]; R=isopropyl)

Nα-[(1S,2R)-2-Methyl-4-oxocyclopentanecarbonyl]-N^{im}-benzyloxycarbonyl-L-histidyl-L-prolineamide (40.0 g) obtained in the same manner as in Example 1 was dissolved in methanol (500 ml), and 10% palladium-carbon (1.0 g) was added followed by catalytic hydrogenation for 6 hours. The catalyst was filtered off on celite and the filtrate was concentrated under reduced pressure to give a residue (32.0 g). The residue was recrystallized from methanol (75 ml) to give Nα-[(1S,2R)-2-methyl-4-oxocyclopentanecarbonyl]-L-histidyl-L-prolineamide (22.5 g) and further recrystallization from isopropanol (68 ml) gave the title compound (22.4 g).

| Elemental analysis: C₁₈H₂₅N₅O₄ · 1 i-C₃H₈OH | | | |
|---|---|---|---|
| Calculated : | C, 57.91; | H, 7.64; | N, 16.08 |
| Found : | C, 57.76; | H, 7.68; | N, 16.35 |

### Example 7

### Nα-[(1S,2R)-2-Methyl-4-oxocyclopentanecarbonyl]-N^{im}-benzoyl-L-histidyl-L-prolineamide (compound [II])

Nα-[(1S,2R)-2-Methyl-4-oxocyclopentanecarbonyl]-L-histidyl-L-prolineamide (3.75 g) was dissolved in N,N'-dimethylformamide (30 ml), and triethylamine (1.21 g) was added, followed by rather gradual addition of benzoyl chloride (1.48 g) with stirring under ice-cooling. The mixture was stirred for 4 hours under ice-cooling. The reaction mixture was poured to dichloromethane (100 ml) and washed with water (100 ml), a saturated aqueous sodium hydrogencarbonate solution (100 ml) and water (100 ml). The dichloromethane layer was dried over anhydrous magnesium sulfate, filtrated and concentrated under reduced pressure to give the title compound (4.41 g, yield 92%) as an oil.
NMR (CDOD₃)δ ppm: 1.01(d,3H,J=0.681Hz), 1.83-2.19(m,7H), 2.52-2.68(m,2H), 2.87-2.96(m,1H), 3.05-3.17(m,2H), 3.53-3.63(m,1H), 3.82-3.93(m,1H), 4.38-4.47(m,1H), 4.86-4.93(m,1H), 7.37-8.19(m,7H)

### Example 8

### Nα-[(1S,2R)-2-Methyl-4-oxocyclopentanecarbonyl]-L-histidyl-L-prolineamide · 1H₂O (compound [III])

Nα-[(1S,2R)-2-Methyl-4-oxocyclopentanecarbonyl]-N^{im}-benzoyl-L-histidyl-L-prolineamide (4.41 g) obtained in the same manner as in Example 7 was dissolved in methanol (30 ml), and the mixture was stirred at room temperature for 3 hours in the presence of Zeolite 3A (2 g). The Zeolite was filtered off, and the filtrate was treated with activated charcoal and concentrated under reduced pressure. The obtained residue was dissolved in water (50 ml). The aqueous layer was washed with dichloromethane (50 ml × 2) and concentrated to 5 ml. Acetonitrile (50 ml) was added and the mixture was left standing at room temperature for 13 hours. The resulting crystals were collected by filtration and dried under reduced pressure at 50°C for 8 hours to give the title compound (2.67 g, yield 71%).

### Industrial Applicability

According to the present invention, a simple method comprising introduction of a protecting group into the histidine moiety of compound [I] obtained by a known method to give compound [II], separating and purifying same in an organic solvent, and hydrogenation or solvolysis for deprotection permits efficient separation and purification of compound [I] from the reaction mixture. The compound [I] thus obtained can be recrystallized easily as a solvate from water and the like, thereby permitting production of solvate [III] at high purity and in high yields.

## Claims

1. A method for separation and purification of a TRH derivative, comprising introducing an imino-protecting group into a histidine moiety of a TRH analog of the following formula [I] 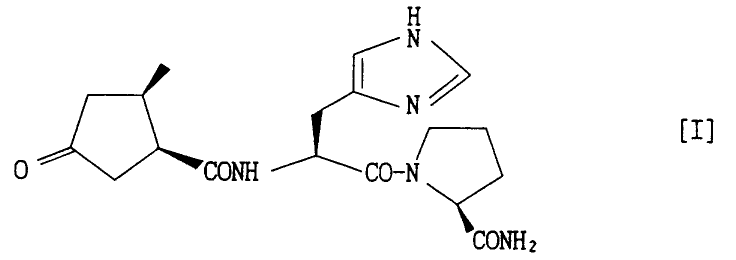 to give an imino-protected compound of the following formula [II] 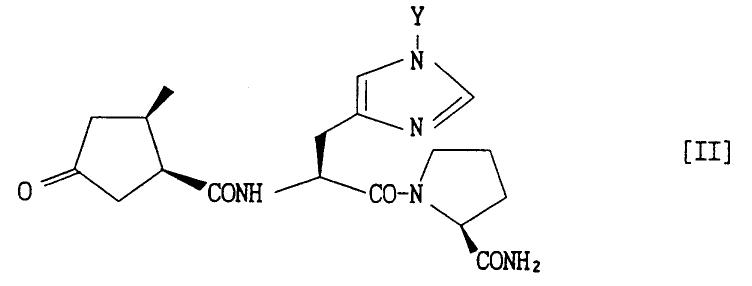 wherein Y is an imino-protecting group, and separating-purifying same in an organic solvent.

2. The method of claim 1, wherein the imino-protecting group is benzyloxycarbonyl group.

3. The method of claim 1, wherein the imino-protecting group is an acyl group.

4. The method of claim 3, wherein the acyl group is benzoyl group.

5. A method for producing a solvate of a TRH derivative, comprising further recrystallizing, from water or a lower alcohol, a compound of the following formula [I] 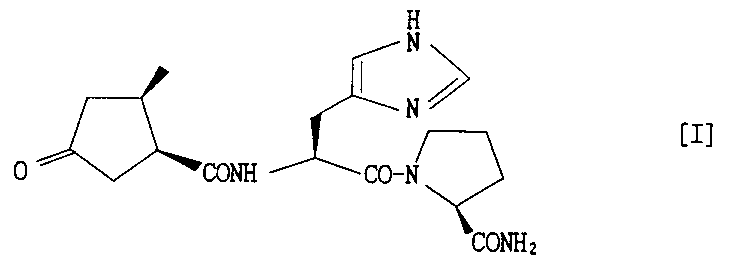 which is obtained by catalytic hydrogenation or solvolysis of the imino-protected compound obtained in claim 1.

6. An Nα-[(1S,2R)-2-methyl-4-oxocyclopentanecarbonyl]-L-histidyl-L-prolineamide hydrate obtained by the method of claim 5.
